# EUROPÄISCHE PATENTSCHRIFT

(11) **EP 1 721 635 B1**
(45) Veröffentlichungstag und Bekanntmachung des Hinweises auf die Patenterteilung: **25.07.2012**
(21) Anmeldenummer: 06009229.3
(22) Anmeldetag: 04.05.2006
(51) Int. Cl.: A61N 5/06, A61B 5/00

(54) **Lichtsystem zur photodynamischen Diagnose und/oder Therapie**
Light system for photodynamic diagnosis and/or therapy
Système d'illumination pour le diagnostic et/ou la thérapie photodynamique

(30) Priorität: 11.05.2005 DE 102005022608
(43) Veröffentlichungstag der Anmeldung: 15.11.2006
(73) Patentinhaber: Karl Storz GmbH & Co. KG, 78532 Tuttlingen (DE)
(72) Erfinder: Irion, Klaus M., 78576 Liptingen (DE); Ehrhardt, André, 78573 Wurmlingen (DE); Schmal, Andreas, 78576 Emmingen-Liptingen (DE)
(74) Vertreter: Weller, Wolfgang

(56) Entgegenhaltungen:
- EP-A- 1 450 190
- DE-A1- 3 444 823
- DE-A1- 19 539 558
- DE-U1- 29 517 716
- US-A- 5 039 191
- US-B1- 6 413 268

## Beschreibung

Die vorliegende Erfindung betrifft ein Lichtsystem zur photodynamischen Diagnose und/oder Therapie, mit einer inkohärenten Lichtquelle und einem eine Eingangsgrenzfläche aufweisenden Lichtleiter, wobei die Eingangsgrenzfläche und die Lichtquelle relativ zueinander positionierbar sind.

Solche Lichtsysteme sind bekannt und werden z.B. von der Anmelderin unter der Bezeichnung T-light entwickelt. Ein solches Lichtsystem ist z.B. in W. Beyer, R. Waidelich, R. Knuechel, H. Stepp, R. Baumgartner, A. Hofstetter: Technical concepts for white light Photodynamic Therapy of bladder cancer. Med. Laser Appl. 17:37-40 (2002), beschrieben.

Solche Lichtsysteme werden z.B. in der photodynamischen Therapie angewendet. Bei dieser Behandlungsmethode werden einem Patienten sog. Photosensitizer verabreicht. Diese Photosensitizer können entweder spezifisch auf malignes Gewebe angewendet werden oder bevorzugter sind es Verbindungen, die sich von Natur aus in malignem Gewebe ansammeln. Werden diese Photosensitizer mit Licht gewisser Wellenlängen bestrahlt, kommt es zu einem phototoxischen Effekt, der das Gewebe, in dem sich die Photosensitizer angesammelt haben, zerstören kann.

Um bei der Behandlung einen maximalen Effekt zu erreichen, muss eine möglichst große Lichtmenge auf das mit dem Photosensitizer behandelten Gewebe angewendet werden. Beim Harnblasenkarzinom erfolgt dieses bspw. über spezielle Katheter, die einen Lichtleiter aufweisen an dessen distalem Ende eine Streustange angeordnet ist, um das am proximalen Ende in den Lichtleiter eingekoppelte Licht über einen möglichst großen Bereich zu verteilen. Damit solche Lichtleiter sterilisierbar sind und nicht zu einer Krankheitsübertragung zwischen Patienten führen, sind diese im Allgemeinen abnehmbar über einen Stecker mit der Lichtquelle verbunden.

Es hat sich nun gezeigt, dass beim Abnehmen und Einsetzen des Steckers, in eine Steckeraufnahme des Lichtsystems diese neu justiert werden muss, um die Eingangsgrenzfläche des Lichtleiters im Fokus der Lichtquelle zu positionieren, um eine möglichst große Lichtmenge in den Lichtleiter einzukoppeln.

Bei den bisher bekannten Systemen erfolgt dies dadurch, dass eine Referenzfaser in der Steckeraufnahme angeordnet wird und die Steckeraufnahme anschließend manuell justiert wird. Diese Referenzfaser wird dann aus der Steckeraufnahme entnommen und durch das proximale Ende des Lichtleiters ersetzt. Dies ist ein äußerst komplizierter und zeitaufwändiger Prozess, der die Zeit, in der ein solches System zur Patientenbehandlung bereitsteht, deutlich verkürzt. Ferner wird zur Justage eines solchen Systems spezialisiertes Personal benötigt, was die Kosten der Verwendung dieses Systems deutlich erhöht.

Aus der US 6,413,268 B1 ist eine Vorrichtung zur UV-Therapie mit einer Basiseinheit bekannt, die eine UV-B-Kurzbogenlampe und einen Anschluss zur Abgabe von UV-B-Strahlung mit einem vorgegebenen Wellenlängenbereich aufweist, wobei an dem Anschluss ein optischer Leiter angeschlossen ist, dessen anderes Ende mit einem Handgriff verbunden ist.

Aus der DE 195 39 558 A1 ist eine Übertragungsvorrichtung für einen Laserstrahl mit einer optischen Faser bekannt, die als Gradientenfaser ausgebildet ist und einen Durchmesser eines Kerns der optischen Faser, eine Brechzahl no an der Mitte des Kerns der optischen Faser und einer Differenz An zwischen den Brechzahlen an der Mitte des Kerns der optischen Faser und an einem Umfangsabschnitt des Kerns der optischen Faser aufweist. Es werden ferner Vorrichtungen und Verfahren zum Fokussieren eines Laserstrahls auf die Einfallseitenebene der optischen Faser beschrieben.

Aus der EP 1 450 190 A1 ist eine Vorrichtung zur Einkopplung von Licht aus einer Licht abgebenden Vorrichtung in eine optische Faser in einer optoelektronischen Vorrichtung bekannt, die einen Mikroaktuator zum Positionieren des Endes der Faser im Hinblick auf die Licht abgebende Vorrichtung sowie eine Kontrollschaltung aufweist, bei der der manipulierbare Wert die Position des Faserendes ist.

Aus der US 5,039,191 ist eine optische Kopplungsvorrichtung bekannt, die Folgendes aufweist: einen optischen Laseremitter, eine optische Faser und Anbringungsmittel zum Anbringen des Lasers relativ zur optischen Faser, um von dem Laser abgegebenes Licht in die optische Faser einzukoppeln, Detektionsmittel zum Detektieren eines Levels eines vorgegebenen Anteils an Licht in der optischen Faser sowie piezoelektronische Bewegungsmittel zum Bewegen des Lasers in orthogonalen Richtungen in Antwort auf die Detektionsmittel, um die Kopplung von von dem Laser abgegebenen Licht in die optische Faser zu erhöhen.

Aus der DE 295 17 716 U1 ist ein Gerät zur photodynamischen Bestrahlung mit einem Gehäuse, einer im Gehäuse angeordneten Lampe, einem die Lampe umgebenden Reflektor, einer im Strahlenweg von Lampe und Reflektor angeordneten Filtereinheit und einer nach der Filtereinheit angeordneten Lichtaustrittsöffnung des Gehäuses bekannt, die eine Dosiereinrichtung zur genauen Dosierung der vom Gerät an einen Patienten abgegebenen Strahlungsenergie aufweist.

Aus der DE 34 44 823 A1 ist eine lösbare Verbindung zwischen einer Lichtleitfaser und einem Lasergerät insbesondere für medizinische Zwecke bekannt, die aus zwei Koppelelementen besteht, wobei in der Lichtaustrittsöffnüng des ersten Koppelelements eine mit diesem verbundene, parallel und/oder senkrecht zur optischen Achse justierbare Einkoppeloptik angeordnet ist, und wobei das erste Koppelelement eine Einrichtung zur Erzeugung einer axialen Kraft auf das zweite Koppelelement aufweist.

Es ist nun Aufgabe der Erfindung ein Lichtsystem zu beschreiben, bei dem die Justierung der Position der Eingangsgrenzfläche des Lichtleiters und der Lichtquelle relativ zueinander deutlich einfacher und schneller zu erreichen ist, wobei sichergestellt bleibt, dass eine möglichst große Lichtmenge an den Applikationsort geleitet wird.

Erfindungsgemäß wird das Problem dadurch gelöst, dass das Lichtsystem ferner Folgendes aufweist, nämlich einen Lichtleistungsmesser, zum Messen der an einem distalen Ende des Lichtleiters abgegebenen Lichtleistung, eine motorgestützte Positioniereinheit zum Positionieren der Eingangsgrenzfläche und der Lichtquelle relativ zueinander, eine Steuereinheit, die die Positioniereinheit in Abhängigkeit von der von dem Lichtleistungsmesser gemessenen Lichtleistung steuert, und eine Bedieneinheit in Form einer Fernbedienung, mit der ein Patient die Lichtleistung am distalen Ende des Lichtleiters zwischen vorgegebenen Werten variieren kann.

Ein solches System ermöglicht es nun, vor der Behandlung eines Patienten, die Lichtleistung am distalen Ende des Lichtleiters auf einfache Weise direkt zu messen. Die gemessene Lichtleistung wird dann zusammen mit der Position der Eingangsgrenzfläche des Lichtleiters und der Lichtquelle relativ zueinander an eine Steuereinheit weitergeleitet, die dann die Position der Eingangsgrenzfläche des Lichtleiters und der Lichtquelle relativ zueinander so verändert, dass eine gewünschte Lichtleistung am distalen Ende des Lichtleiters erreicht wird. Dieser Vorgang läuft vollkommen automatisiert ab und kann in kurzer Zeit durchgeführt werden, wodurch das Lichtsystem deutlich häufiger einsetzbar ist, als ein Lichtsystem, das eine manuelle Justage benötigt. Außerdem ist diese Justage auch ohne spezialisiertes Personal durchzuführen.

Ein solches Lichtsystem kann also deutlich häufiger und kostengünstiger eingesetzt werden.

Der Begriff "distales Ende des Lichtleiters" wie er zuvor und nachstehend verwendet wird, ist dabei nicht auf die distale Grenzfläche des Lichtleiters beschränkt, sondern schließt auch ggf. an dieser Grenzfläche angebrachte Lichtapplikatoren wie bspw. Streustangen ein.

In einer Ausgestaltung der Erfindung ist mit der Steuereinheit die Lichtleistung am distalen Ende des Lichtleiters maximierbar.

Es ist zwar möglich auch andere Werte für die gewünschte Lichtleistung festzulegen, bevorzugterweise wird aber angestrebt am distalen Ende des Lichtleiters eine möglichst hohe Lichtleistung zu erreichen. Durch diese Maßnahme kann dieses automatisch erreicht werden.

Das Maximieren der Lichtleistung erfolgt dabei im Allgemeinen so, dass die Positioniereinheit aufeinander folgend, die Lichtquelle und die Eingangsfläche des Lichtleiters relativ zueinander in drei Raumrichtungen in einem vorher definierten Bereich verfährt und jeweils das Lichtleistungsmaximum in der Raumrichtung bestimmt. Da es sich bei der Lichtleistungsverteilung um eine gauß-förmige Verteilung handelt, stellt die Position des dritten Maximums dabei das gesamte Lichtleistungsmaximum dar.

In einer Ausgestaltung der zuvor genannten Maßnahme erfolgt ein Maximieren der Lichtleistung am distalen Ende des Lichtleiters durch einen Gradienten-Optimierungsverfahren.

Bei einem Gradienten-Optimierungsverfahren erfolgt das Verfahren der Lichtquelle und der Eingangsgrenzfläche des Lichtleiters relativ zueinander in den drei Raumrichtungen nicht äquidistant, sondern entsprechend dem aktuellen Lichtleistungsgradienten. Somit wird das Lichtleistungsmaximum deutlich schneller erreicht.

In einer weiteren Ausgestaltung der Erfindung, ist zwischen der Lichtquelle und dem Lichtleiter ein IR-Blockfilter vorgesehen.

Inkohärente Lichtquellen geben neben dem für die photodynamische Therapie benötigten weißen Licht, auch Strahlung im IR-Bereich (Infrarotbereich), also Wärmestrahlung, ab. Ein kontinuierliches Einwirken dieser Wärmestrahlung auf das proximale Ende des Lichtleiters kann dort zu Verformungen führen, die die Effizienz des Einkoppelns von Licht in das proximale Ende des Lichtleiters deutlich herabsetzen.

Das IR-Blockfilter filtert nun aus dem von der Lichtquelle abgegebenen Licht die Wärmestrahlung zum Teil oder vollständig heraus und leitet diese an eine andere Stelle ab, wodurch das proximale Ende des Lichtleiters keiner Wärmebelastung mehr ausgesetzt ist. Dadurch kann die Lebensdauer eines Lichtleiters deutlich erhöht und die Effizienz des Einkoppelns von Licht deutlich verbessert werden.

In einer Ausgestaltung der zuvor genannten Maßnahme ist ein Detektor vorgesehen, der von dem IR-Blockfilter reflektierte Strahlung detektiert.

Die Verwendung eines Detektors in Kombination mit einem IR-Blockfilter kann dazu verwendet werden, die Intensität der von der Lichtquelle abgegebenen Strahlung zu messen, ohne dass dabei für die Therapie nützliches Weißlicht aus dem Licht ausgekoppelt werden muss.

Bei der Lichtquelle kann die Lichtleistung z.B. lebensdauerbedingt abfallen, was sich auch in der Leistung der IR-Strahlung niederschlägt. Wird dieses durch den Detektor festgestellt, kann z.B. die Ausgangsspannung eines Netzteils der Lichtquelle erhöht werden, um damit die Leistung auf den ursprünglichen Wert hochzufahren.

Ist dieses nicht mehr möglich, kann die Information über die Lichtleistung z.B. dazu verwendet werden, um in Abhängigkeit von dem Abfall der Lichtleistung die Bestrahlungsdauer zu verlängern, um jeweils die benötigte Strahlendosis zu verabreichen.

Ein solcher Detektor kann außerdem auf einfache Weise feststellen, ob die Lichtquelle noch funktionsfähig ist.

In einer weiteren Ausgestaltung dieser Maßnahme ist die Leistung der Lichtquelle in Abhängigkeit von der durch den Detektor detektierten Strahlung steuerbar.

Durch diese Maßnahme kann bei einem detektierten Leistungsabfall der Lichtquelle, z.B. über die Lebensdauer, die Leistung der Lichtquelle automatisch, z.B. über die Ausgangsspannung eines Netzteils der Lichtquelle erhöht werden, wodurch sichergestellt wird, dass die Lichtquelle immer Licht der gleichen Leistung abgibt.

In einer weiteren Ausgestaltung der Erfindung wird die Lichtleistung am distalen Ende des Lichtleiters langsam auf einen Maximalwert erhöht.

Es hat sich gezeigt, dass wenn eine Bestrahlung bei einem Patienten vorgenommen wird, das Schmerzempfinden, das bei einer abrupten Applikation der Maximalbestrahlung auftreten kann, deutlich reduziert wird, wenn die Leistung am distalen Ende des Lichtleiters langsam auf die maximale Bestrahlungsstärke erhöht wird.

In einer weiteren Ausgestaltung der Erfindung erfolgt die Steuerung der Lichtleistung am distalen Ende des Lichtleiters durch Steuern der Leistung der Lichtquelle.

Durch diese Maßnahme, die z.B. durch einfaches Verändern der Ausgangsspannung eines Netzteils der Lichtquelle erreicht werden kann, ist ein besonders einfaches Steuern der Lichtleistung am distalen Ende des Lichtleiters möglich.

In einer weiteren Ausgestaltung der Erfindung erfolgt die Steuerung der Lichtleistung am distalen Ende des Lichtleiters durch Steuern der Position der Eingangsgrenzfläche des Lichtleiters und der Lichtquelle relativ zueinander.

Die Steuerung der Leistung der Lichtquelle z.B. über die Spannung ist nur in begrenzten Bereichen möglich, da z.B. bei einer zu geringen Spannung der Durchbruch zusammenbricht und kein Licht mehr abgegeben wird. Durch die oben genannte Maßnahme ist es nun möglich am distalen Ende des Lichtleiters auch eine Lichtleistung zu erreichen, die unterhalb der Leistung liegt, die alleine durch Steuern der Leistung der Lichtquelle möglich ist.

In einer weiteren Ausgestaltung ist mit der Lichtquelle gepulstes Licht abgebbar.

Untersuchungen haben gezeigt, dass eine fraktionierte Bestrahlung, d.h. eine Bestrahlung mit definierten Unterbrechungen, den therapeutischen Effekt der photodynamischen Therapie erhöht. Vorteilhafterweise liegt dabei die Frequenz des gepulsten Lichts unterhalb der Ansprechzeit von Schmerzzellen, wodurch das Schmerzempfinden eines Patienten weiter reduziert werden kann.

In einer Ausgestaltung der zuvor genannten Maßnahme ist an der Lichtquelle eine Shutterblende zur Erzeugung von gepulstem Licht vorgesehen.

Durch diese Maßnahme ist es möglich die Lichtquelle kontinuierlich zu betreiben, während sich die Shutterblende öffnet und schließt, wodurch das gepulste Licht erzeugt wird. Dies ist für die Lichtquelle deutlich weniger belastend als bspw. ein schnelles Ein- und Ausschalten. Dadurch wird die Lebensdauer der Lichtquelle deutlich erhöht.

In einer weiteren Ausgestaltung der Erfindung ist eine Eingabeeinheit vorgesehen, mit der eine von dem distalen Ende des Lichtleiters abzugebende Strahlungsdosis festlegbar ist, wobei insbesondere mit der Eingabeeinheit in Abhängigkeit von der Leistung der Lichtquelle, der Position der Eingangsgrenzfläche des Lichtleiters und der Lichtquelle relativ zueinander und der abzugebenden Strahlungsdosis eine Bestrahlungszeit berechenbar und einstellbar ist.

Diese Maßnahme vereinfacht den Betrieb eines Lichtsystems deutlich, da nur noch ein einziger Wert eingegeben werden muss und alle anderen Werte von dem Lichtsystem selbst berechnet werden.

In einer weiteren Ausgestaltung der zuvor genannten Maßnahme erfolgt die Berechnung der Bestrahlungszeit kontinuierlich.

Während des Betriebs eines Lichtsystems kann es z.B. durch ein Aufheizen der Lichtquelle, oder durch Eingriff des Patienten oder eines Operateurs, zu Veränderungen der am distalen Ende des Lichtleiters abgegebenen Leistung kommen. Durch die kontinuierliche Berechnung wird die Bestrahlungszeit während der gesamten Bestrahlung automatisch diesen Veränderungen angepasst.

In einer weiteren Ausgestaltung der Erfindung ist ein Timer vorgesehen mit dem nach Ablauf der Bestrahlungszeit die Lichtquelle automatisch abschaltbar ist.

Durch diese Maßnahme wird ein Operateur deutlich entlastet, da dieser nur noch den Lichtleiter am Patienten anbringen und das Lichtsystem einschalten muss. Es ist kein gesondertes Nachprüfen, ob die Bestrahlungszeit abgelaufen ist, mehr notwendig.

In einer weiteren Ausgestaltung der Erfindung weist die motorgestützte Positioniereinheit zumindest einen Linearmotor auf.

Aufgrund ihrer kompakten Bauweise und ihrer hohen Präzision, sind Linearmotoren besonders für den Einsatz in einer solchen Positioniereinheit geeignet.

In einer weiteren Ausgestaltung der Erfindung ist am distalen Ende des Lichtleiters eine Schutzabdeckung vorgesehen, wobei diese Schutzkappe sterilisierbar und/oder zur Einmalverwendung vorgesehen ist.

Aufgrund der in dem Lichtleistungsmesser vorhandenen Elektronik ist dieser häufig nur schwer oder gar nicht zu sterilisieren. Am distalen Ende des Lichtleiters kann nun für die Messung eine Schutzabdeckung angeordnet werden, die verhindert, dass das distale Ende, das zum Anbringen am Patienten steril sein muss, durch den Lichtleistungsmesser kontaminiert wird. Die Schutzabdeckung kann dabei sterilisierbar und somit wiederverwendbar ausgebildet sein oder zur Einmalverwendung vorgesehen und einfach nach Verwendung weggeworfen werden.

In einer weiteren Ausgestaltung der Erfindung weist das Lichtsystem ferner ein die Lichtquelle enthaltendes Beleuchtungsmodul mit einer Steckeraufnahme auf, wobei ein proximales Ende des Lichtleiters abnehmbar in der Steckeraufnahme angeordnet ist.

Durch diese Maßnahme kann der Lichtleiter von der Lichtquelle abgenommen werden und z.B. getrennt davon sterilisiert werden. Hierdurch kann die Sterilität des Lichtleiters, der im Patienten angeordnet wird sichergestellt werden, ohne dass die empfindlichen elektronischen Bauteile den Belastungen einer Sterilisation ausgesetzt werden.

Es versteht sich, dass die zuvor genannten und die nachstehend noch zu nennenden Merkmale nicht nur in der angegebenen Kombination, sondern auch in anderen Kombinationen oder in Alleinstellung anwendbar sind, ohne den Rahmen der vorliegenden Erfindung zu verlassen.

Ausführungsbeispiele der Erfindung sind in den Zeichnungen dargestellt und werden in der nachfolgenden Beschreibung näher erläutert. Es zeigen:
- Fig. 1: stark schematisch ein Lichtsystem zur photodynamischen Diagnose und/oder Therapie,
- Fig. 2: schematisch eine weitere Ausführungsform eines Lichtsystems zur photodynamischen Diagnose und/oder Therapie,
- Fig. 3: ein Beleuchtungsmodul eines Lichtsystems, und
- Fig. 4: eine Positioniereinheit des Beleuchtungsmoduls von Fig. 3.

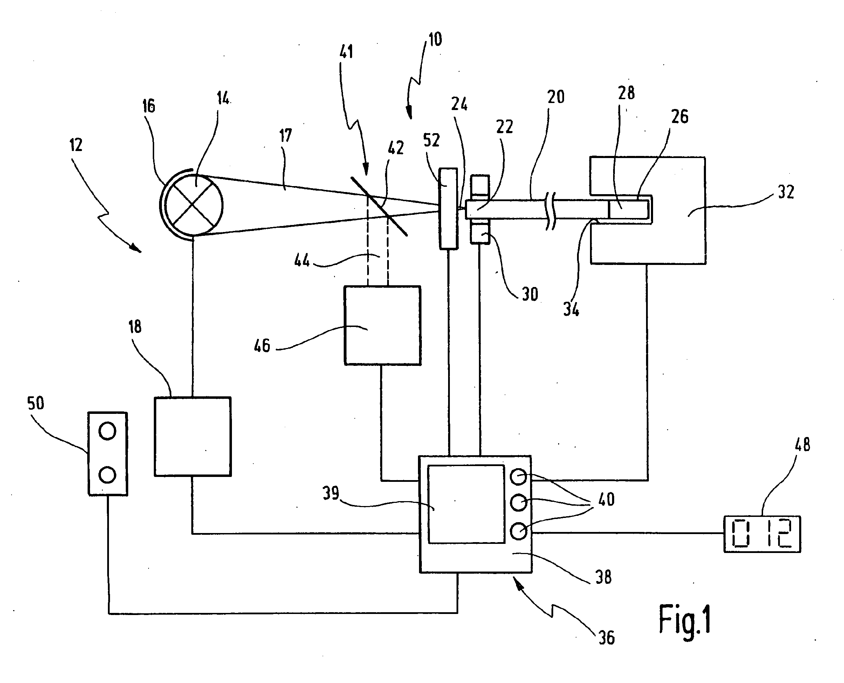

In Fig. 1 ist ein Lichtsystem in seiner Gesamtheit mit der Bezugsziffer 10 bezeichnet.

Das Lichtsystem 10 weist eine Lichtquelle 12 auf, die hier aus einer Xenon-Kurzbogenlampe 14 besteht, die von einem Reflektor 16 umgeben ist, um einen gebündelten inkohärenten Lichtstrahl 17 zu bilden. Die Lichtquelle 12 weist ferner ein Netzgerät 18 für die Xenon-Kurzbogenlampe 14 auf.

Das Lichtsystem 10 weist ferner einen Lichtleiter 20 mit einem proximalen Ende 22 an dem sich eine Eingangsgrenzfläche 24 befindet, und einem distalen Ende 26 auf, an dem eine Streustange 28 angeordnet ist. Diese Streustange 28 dient dazu, das an der Eingangsgrenzfläche 24 in den Lichtleiter 20 eingekoppelte Licht, das zum distalen Ende 26 weitergeleitet wird, über einen möglichst großen Bereich zu verteilen.

Das proximale Ende 22 des Lichtleiters 20 ist dabei in einer motorgestützten Positioniereinheit 30 angeordnet, während das distale Ende 26 in einen Lichtleistungsmesser 32 eingeführt ist. Dieser Lichtleistungsmesser 32 weist einen Hohlraum 34 auf, in den das distale Ende 26 des Lichtleiters 20 eingeführt werden kann. In diesem Hohlraum 34 sind hier nicht dargestellte Sensoren angeordnet, die die am distalen Ende 26 des Lichtleiters 20 abgegebene Lichtleitung messen. Der Lichtleistungsmesser 32 und die Positioniereinheit 30 sind mit einer Steuereinheit 36, die hier durch den Mikrokontroller 38 gebildet wird, verbunden.

Der Lichtleistungsmesser 32 misst nun die am distalen Ende 26 des Lichtleiters 20 abgegebene Lichtleistung und leitet diesen Wert an den Mikrokontroller 38 weiter. Der Mikrokontroller 38 steuert nun die Positioniereinheit 30 an, um die Position des proximalen Endes 22 des Lichtleiters 20 und damit der Eingangsgrenzfläche 24 und der Lichtquelle 12 relativ zueinander zu verändern und misst dabei kontinuierlich die Veränderung der Lichtleistung am distalen Ende 26 des Lichtleiters 20. Wenn diese in einer Raumrichtung z.B. in x-Richtung ein Maximum erreicht hat, wird die Bewegung in dieser Raumrichtung eingestellt und eine Bewegung in eine andere Raumrichtung, z.B. in y-Richtung, durchgeführt. Wenn in der dritten Raumrichtung, z.B. in z-Richtung, das Maximum erreicht ist, ist auch das Gesamtmaximum der Lichtleistung am distalen Ende 26 des Lichtleiters 20 erreicht.

Der Mikrokontroller 38 weist ferner einen Bildschirm 39, der hier als Touchscreen ausgebildet ist, und Drehknöpfe 40 auf.

Der Bildschirm 39 kann sowohl dazu dienen, Daten wie die augenblickliche Lichtleistung am distalen Ende 26 des Lichtleiters 20 darzustellen, als auch, da es sich um ein Touchscreen handelt, z.B. besondere Betriebsmodi, wie ein langsames Hochfahren auf einen Maximalwert, auszuwählen. Die Drehknöpfe 40 dienen ebenfalls der Bedienung des Mikrokontrollers 38 und bilden zusammen mit dem Touchscreen eine Eingabeeinheit.

Das Lichtsystem 10 weist ferner ein IR-Blockfilter 41 auf, das hier als teildurchlässiger Spiegel 42 ausgebildet ist. Dieser teildurchlässige Spiegel 42 ist im Lichtstrahl 17 der Lichtquelle 12 angeordnet und reflektiert einen IR-Anteil 44 des Lichtstrahls 17, der hier mit gestrichelten Linien dargestellt ist, in Richtung eines Detektors 46.

Bei dem teildurchlässigen Spiegel 42 handelt es sich um einen sog. kalten Spiegel, der sichtbares Licht durchlässt und IR-strahlung reflektiert. Dadurch führt das durch den Spiegel 42 durchtretende sichtbare Licht zu einer deutlich geringeren Erwärmung.

Der Detektor stellt nun die Leistung der von der Lichtquelle 12 abgegebenen IR-Strahlung 44 fest und leitet dieses Signal an den Mikrokontroller 38 weiter. Dieses Signal kann ebenfalls auf dem Bildschirm 39 dargestellt werden und informiert einen Operateur z.B. über den Zustand der Xenon-Kurzbogenlampe 14. Sollte nun die Leistung der Xenon-Kurzbogenlampe 14 im Laufe der Zeit abfallen, kann der Mikrokontroller 38 das Netzteil 18 ansteuern, um die Ausgangsspannung für die Xenon-Kurzbogenlampe 14 zu erhöhen, wodurch die Leistung der xenon-Kurzbogenlampe 14 wieder auf ihren ursprünglichen Wert erhöht wird. Ist ein weiteres Erhöhen der Ausgangsspannung nicht mehr möglich, berechnet der Mikrokontroller 38 anhand des neuen Leistungswerts eine Veränderung der Bestrahlungszeit und gibt diese an einen Timer 48 weiter. Wenn der Timer 48 einen vorgegebenen Wert, also z.B. das Ende der Bestrahlungszeit erreicht hat, gibt dieser ein Signal an den Mikrokontroller 38 weiter, der dann mittels des Netzteils 18 die Xenon-Kurzbogenlampe 14 abschaltet.

Das Lichtsystem 10 weist ferner eine Bedieneinheit 50 auf, mit der z.B. ein Patient die Lichtleistung am distalen Ende 26 des Lichtleiters 20 innerhalb von vorgegebenen Parameter variieren kann. Er kann z.B. die Lichtleistung herabsetzen, wenn durch die Bestrahlung ein erhöhtes Schmerzempfinden verursacht wird. Er kann ferner die Lichtleistung erhöhen, wodurch die Bestrahlungszeit verkürzt wird.

Der Mikrokontroller 38 registriert wiederum die durch einen Patienten vorgenommenen Veränderungen in der Lichtleistung und berechnet daraus kontinuierlich eine neue Bestrahlungszeit, die wiederum an den Timer 48 weitergegeben wird.

Ferner weist das Lichtsystem 10 eine Shutterblende 52 auf, die zwischen der Lichtquelle 12 und der Eingangsgrenzfläche 24 des Lichtleiters 20 angeordnet ist. Durch diese Shutterblende 52 kann das auf die Eingangsgrenzfläche 24 des Lichtleiters 20 auftreffende Licht gepulst werden, was zu einer erhöhten therapeutischen Effizienz führt. Die Shutterblende 52 ist ebenfalls mit dem Mikrokontroller 38 verbunden und der Mikrokontroller 38 berechnet anhand der Pulsfrequenz der Shutterblende 52 die Bestrahlungszeit und leitet diese wiederum an den Timer 48 weiter. Die Drehknöpfe 40 des Mikrokontrollers 38 können dabei z.B. dazu verwendet werden, die Pulsfrequenz der Shutterblende zu regulieren.

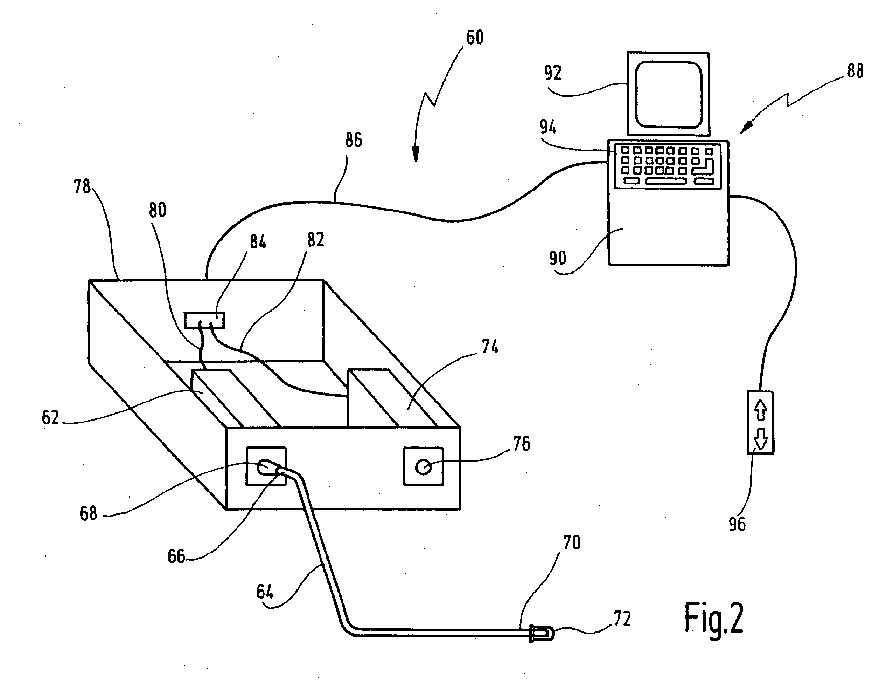

In Fig. 2 ist ein Lichtsystem in seiner Gesamtheit mit der Bezugsziffer 60 bezeichnet.

Dieses Lichtsystem 60 weist ein Beleuchtungsmodul 62 und einen Lichtleiter 64 auf. Ein proximales Ende 66 des Lichtleiters 64 ist dabei lösbar in einer Steckeraufnahme 68 des Beleuchtungsmoduls 62 angeordnet. Dieses Beleuchtungsmodul 62 weist eine hier nicht dargestellte inkohärente Lichtquelle auf, wobei es sich z.B. um die Lichtquelle 12 handeln kann, mit der Licht in den in der Steckeraufnahme 68 angeordneten Lichtleiter 64 eingekoppelt werden kann. An einem distalen Ende 70 des Lichtleiters 64 ist eine transparente Schutzabdeckung 72 angeordnet, die das distale Ende 70 vor einer Kontamination während der Lichtleistungsmessung schützt. Diese Schutzabdeckung 72 ist dabei optisch vollständig transparent um eine Verfälschung der Messergebnisse zu vermeiden. Die Schutzabdeckung 72 ist zur Einmalverwendung ausgelegt und wird nach Gebrauch weggeworfen.

Das Lichtsystem 60 weist ferner einen Lichtleistungsmesser 74 auf, der eine Buchse 76 aufweist. In diese Buchse 76 kann nun das von der Schutzabdeckung 72 geschützte distale Ende 70 des Lichtleiters 64 eingeführt werden und durch den Lichtleistungsmesser kann die am distalen Ende 70 des Lichtleiters 64 abgegebene Lichtleistung, gemessen werden. Das Beleuchtungsmodul 62 und der Lichtleitungsmesser 74 sind hier in einem Gehäuse 78 vereinigt, wobei die obere Abdeckung des Gehäuses 78 hier nicht dargestellt ist.

Eine Signalleitung 80 des Beleuchtungsmoduls 62 und eine Signalleitung 82 des Lichtleistungsmessers 74 werden in einem Stecker 84 an dem Gehäuse 78 vereinigt und sind mittels eines Kabels 86 mit einer Steuereinheit 88 verbunden.

Die Steuereinheit 88 wird hier durch einen Personal Computer 90 gebildet, der einen Bildschirm 92 und eine alphanumerische Tastatur 94 als Eingabeeinheit aufweist. Die Steuereinheit 88 weist ferner eine Bedieneinheit 96 auf, mit der die am distalen Ende 70 des Lichtleiters 64 abgegebene Strahlung von einem Patienten individuell eingestellt werden kann.

Die Behandlung eines Patienten erfolgt nun wie folgt. Das distale Ende 70 des Lichtleiters 64 wird mit einer Schutzabdeckung 72 versehen und in die Buchse 76 des Lichtleistungsmessers 74 eingeführt. Der Lichtleistungsmesser 74 misst die am distalen Ende 70 des Lichtleiters 64 abgegebene Lichtleistung und übermittelt diese an die Steuereinheit 88. Das Beleuchtungsmodul 62 übermittelt die relative Position des proximalen Endes 66 des Lichtleiters 64 und der hier nicht dargestellten Lichtquelle zueinander an die Steuereinheit 88. Die Steuereinheit 88 steuert nun das Beleuchtungsmodul 62 an und verändert die Position des proximalen Endes 66 des Lichtleiters 64 und der Lichtquelle relativ zueinander in einer Raumrichtung. Gleichzeitig wird durch den Lichtleistungsmesser 74 der aktuelle Lichtleistungsgradient des am distalen Ende 70 abgegebenen Lichts gemessen. Gemäß dieses gemessenen Lichtleistungsgradienten, verändert die Steuereinheit 88 die Distanz mit der die relative Position des proximalen Endes 66 des Lichtleiters 64 und der Lichtquelle zueinander verändert wird, bis in den jeweiligen Raumrichtungen das Maximum erreicht ist.

Nach Abschluss dieses Justagevorgangs wird das distale Ende 70 des Lichtleiters 64 aus der Buchse 76 des Lichtleistungsmessers 74 entnommen und die Schutzabdeckung 72 vom distalen Ende 70 des Lichtleiters 64 entfernt.

Das distale Ende 70 des Lichtleiters 64 wird nun an einem zu behandelnden Patienten angeordnet und ein Operateur gibt mittels der Tastatur 94 eine Maximalbestrahlungsleistung, und eine Minimalbestrahlungsleistung, sowie eine gewünschte Bestrahlungsdosis ein und startet den Bestrahlungsvorgang.

Die Steuereinheit 88 erhöht nun langsam die am distalen Ende 70 des Lichtleiters 64 abgegebene Lichtleistung, bis der gewünschte Maximalwert erreicht wird und berechnet aus den Lichtleistungsdaten und der gewünschten Bestrahlungsdosis die benötigte Bestrahlungszeit. Während der Behandlung kann nun ein Patient über die Bedieneinheit 96, z.B. wenn die Behandlung zu schmerzhaft wird, die abgegebene Lichtleistung reduzieren. Die Steuereinheit 88 registriert diese Veränderung der Lichtleistung und berechnet daraus eine neue Bestrahlungszeit. Nach Ende der berechneten Bestrahlungszeit, das durch einen in der Steuereinheit 88 integrierten Timer ermittelt wird, wird die Lichtquelle des Beleuchtungsmoduls 62 abgeschaltet.

Es versteht sich, dass die für die Ausführungsbeispiele der Fig. 1 und 2 gezeigten Merkmale, insofern diese sich nicht gegenseitig ausschließen, auch in dem jeweilig anderen Anwendungsbeispiel zur Anwendung kommen können.

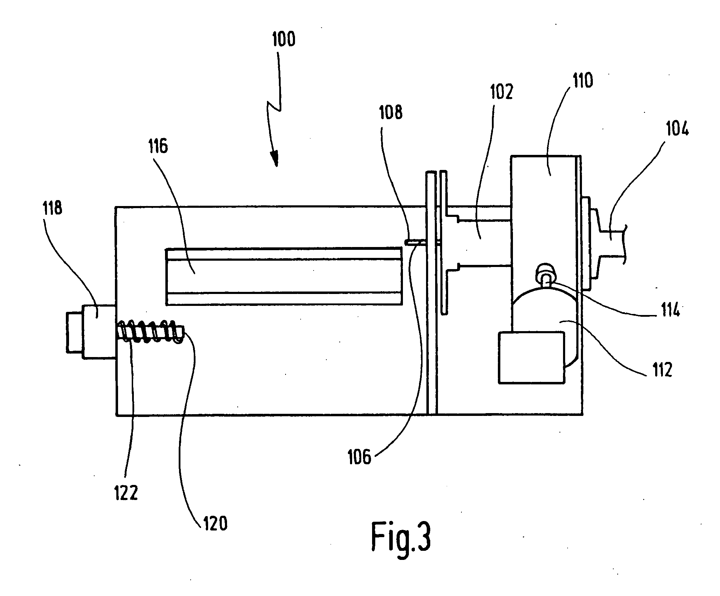

In Fig. 3 ist ein Beleuchtungsmodul wie es in einem Lichtsystem von Fig. 2 verwendet werden kann, in seiner Gesamtheit mit der Bezugsziffer 100 bezeichnet.

Aus Gründen der besseren Verständlichkeit ist die Lichtquelle selbst in dieser Darstellung nicht dargestellt.

Das Beleuchtungsmodul 100 weist eine Steckeraufnahme 102 auf, in der ein Stecker 104 eines Lichtleiters 106 eingebracht wurde, wobei ein proximales Ende 108 des Lichtleiters 106 über die Steckeraufnahme 102 hinausragt.

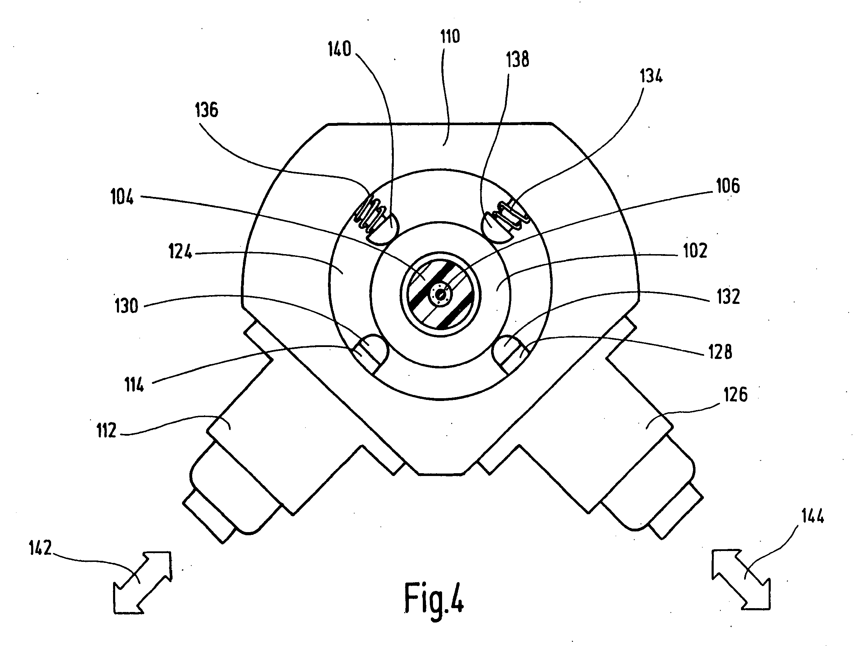

Die Steckeraufnahme 102 ist beweglich in einer Positioniereinheit 110 angeordnet. Diese Positioniereinheit 110 weist zwei im Winkel von 90° zueinander angeordnete Linearmotoren auf, von denen hier nur der Linearmotor 112 sichtbar ist. Dieser Linearmotor 112 weist eine Achse 114 auf, die in die Positioniereinheit 110 hineingeschoben oder aus dieser herausgezogen werden kann und dadurch die Position der Steckeraufnahme 102 verändert. Der genaue Aufbau dieser Positioniereinheit 110 ist im größeren Detail in Fig. 4 dargestellt.

Das Beleuchtungsmodul 100 weist ferner einen weiteren Linearmotor 118 auf, der eine Achse 120 aufweist, die eine Feder 122 aufweist.

Eine in dieses Beleuchtungsmodul 100 einzusetzende Lichtquelle ist entlang einer Schiene 116 verschiebbar angeordnet, wobei die Achse 120 des Linearmotors 118 gegen die Lichtquelle zu liegen kommt und die Feder 122 ebenfalls mit der Lichtquelle verbunden ist.

In diesem Beleuchtungsmodul 100 ist nun die Positioniereinheit 110 für die Bewegung des Lichtleiters 106 in x- und y-Richtung relativ zur inkohärenten Lichtquelle verantwortlich. Die relative Positionsverlagerung zwischen der Lichtquelle und dem proximalen Ende 108 des Lichtleiters 106 in z-Richtung erfolgt in dieser Ausführungsform durch ein Verschieben der Lichtquelle entlang der Schiene 116, was durch den Linearmotor 118 bzw. die Feder 122 erfolgt.

In Fig. 4 ist die Positioniereinheit 110 des Beleuchtungsmoduls 100 von Fig. 3 im größeren Detail dargestellt.

Die Positioniereinheit 110 weist in ihrer Mitte eine kreisförmige Öffnung 124 auf, in der die Steckeraufnahme 102 gelagert ist. In dieser Steckeraufnahme 102 sind der Stecker 104 nebst dem darin angeordneten Lichtleiter 106 angeordnet.

Die Positioniereinrichtung weist nun neben dem in Fig. 3 dargestellten Linearmotor 112 einen weiteren Linearmotor 126 auf, der im Winkel von 90° zum Linearmotor 112 angeordnet ist. Die Achse 114 des Motors 112 und eine Achse 128 des Motors 126 reichen dabei in die Öffnung 124 der Positioniereinheit 110 hinein. Die beiden Achsen 114 und 128 sind ebenfalls in einem Winkel von 90° zueinander angeordnet.

Am äußersten Ende der Achsen 114 und 128 sind Achsenkappen 130 und 132 angeordnet, die hier in Form von Halbkugeln ausgebildet sind. Die Steckeraufnahme 102 kommt dabei auf den Achskappen 130 und 132 zu liegen. Jeweils gegenüber den jeweiligen Motorachsen 114, 128 bzw. Achskappen 130, 132 sind Federn 134 und 136 angeordnet, an deren Ende Federkappen 138 und 140 angeordnet sind. Die Federn 134, 136 drücken die Federkappen 138, 140 gegen die Steckeraufnahme 102, wodurch diese gegen die jeweils gegenüber liegende Achskappe 130 bzw. 132 gedrückt wird.

Die Linearmotoren 112, 126 können nun die Motorachsen 114, 128 jeweils in Richtung der Doppelpfeile 142 bzw. 144 verschieben. Bei einer Bewegung der Motorachsen 114, 128 in Richtung der jeweiligen Linearmotoren 112, 126, stellen die Federn 134, 136 eine Rückstellkraft zur Verfügung, so dass die Steckeraufnahme 102 jeweils an die Achskappen 132, 130 gedrückt bleibt. Durch die Bewegung der Motorachsen 114, 128 in Richtung der Doppelpfeile 142, 144, kann nun die Steckeraufnahme 102 in einer beliebigen x/y-Position in der Öffnung 124 der Positioniereinheit angeordnet werden, wodurch die Positionierung des Lichtleiters 106 gegenüber der Lichtquelle in zwei Dimensionen erreicht wird.

## Patentansprüche

1. Lichtsystem zur photodynamischen Diagnose und/oder Therapie, mit einer inkohärenten Lichtquelle (12) und mit einem eine Eingangsgrenzfläche (24) aufweisenden Lichtleiter (20; 64; 106), wobei die Eingangsgrenzfläche (24) und die Lichtquelle (12) relativ zueinander positionierbar sind, **dadurch gekennzeichnet, dass** das Lichtsystem (10; 60) ferner Folgendes aufweist, nämlich
einen Lichtleistungsmesser (32; 74) zum Messen der an einem distalen Ende (26; 70) des Lichtleiters (20; 64; 106) abgegebenen Lichtleistung,
eine motorgestützte Positioniereinheit (30; 110) zum Positionieren der Eingangsgrenzfläche (24) und der Lichtquelle (12) relativ zueinander,
eine Steuereinheit (36; 88), die die Positioniereinheit (30; 110) in Abhängigkeit der vom Lichtleistungsmesser (32; 74) gemessenen Lichtleistung steuert, und eine Bedieneinheit (50; 96) in Form einer Fernbedienung, mit der durch einen Patienten die Lichtleistung am distalen Ende (26; 70) des Lichtleiters (20; 64; 106) zwischen vorgegebenen Werten variier bar ist.

2. Lichtsystem nach Anspruch 1, **dadurch gekennzeichnet, dass** mit der Steuereinheit (36; 88) die Lichtleistung am distalen Ende (26; 70) des Lichtleiters (20; 64; 106) maximierbar ist.

3. Lichtsystem nach Anspruch 2, **dadurch gekennzeichnet, dass** ein Maximieren der Lichtleistung am distalen Ende (26; 70) des Lichtleiters (20; 64; 106) durch ein Gradienten-Optimierungsverfahren erfolgt.

4. Lichtsystem nach einem der Ansprüche 1 bis 3, **dadurch gekennzeichnet, dass** zwischen der Lichtquelle (12) und dem Lichtleiter (20; 64; 106) ein IR-Blockfilter (41) vorgesehen ist.

5. Lichtsystem nach Anspruch 4, **dadurch gekennzeichnet, dass** ein Detektor (46) vorgesehen ist, der von dem IR-Blockfilter (41) reflektierte Strahlung (44) detektiert.

6. Lichtsystem nach Anspruch 5, **dadurch gekennzeichnet, dass** die Leistung der Lichtquelle (12) in Abhängigkeit von der durch den Detektor (46) detektierten Strahlung (44) steuerbar ist.

7. Lichtsystem nach einem der Ansprüche 2 bis 6, **dadurch gekennzeichnet, dass** die Lichtleistung am distalen Ende (26; 70) des Lichtleiters (20; 64; 106) langsam auf einen Maximalwert erhöht wird.

8. Lichtsystem nach einem der Ansprüche 1 bis 7, **dadurch gekennzeichnet, dass** die Steuerung der Lichtleistung am distalen Ende (26; 70) des Lichtleiters (20; 64; 106) durch Steuern der Leistung der Lichtquelle (12) erfolgt.

9. Lichtsystem nach einem der Ansprüche 1 bis 7, **dadurch gekennzeichnet, dass** die Steuerung der Lichtleistung am distalen Ende (26; 70) des Lichtleiters (20; 64; 106) durch Steuern der Position der Eingangsgrenzfläche (24) des Lichtleiters (20; 64; 106) und der Lichtquelle (12) relativ zueinander erfolgt.

10. Lichtsystem nach einem der Ansprüche 1 bis 9, **dadurch gekennzeichnet, dass** mit der Lichtquelle (12) gepulstes Licht abgebbar ist.

11. Lichtsystem nach Anspruch 10, **dadurch gekennzeichnet, dass** an der Lichtquelle (12) eine Shutterblende (52) zur Erzeugung von gepulstem Licht vorgesehen ist.

12. Lichtsystem nach einem der Ansprüche 1 bis 11, **dadurch gekennzeichnet, dass** eine Eingabeeinheit vorgesehen ist, mit der eine von dem distalen Ende (26; 70) des Lichtleiters (20; 64; 106) abzugebende Strahlungsdosis festlegbar ist.

13. Lichtsystem nach Anspruch 12, **dadurch gekennzeichnet, dass** mit der Eingabeeinheit in Abhängigkeit von der Leistung der Lichtquelle (12), der Position der Eingangsgrenzfläche (24) des Lichtleiters (20; 64; 106) und der Lichtquelle (12) relativ zueinander und der abzugebenden Strahlungsdosis eine Bestrahlungszeit berechenbar und einstellbar ist.

14. Lichtsystem nach Anspruch 13, **dadurch gekennzeichnet, dass** die Berechnung der Bestrahlungszeit kontinuierlich erfolgt.

15. Lichtsystem nach einem der Ansprüche 1 bis 14, **dadurch gekennzeichnet, dass** ein Timer (48) vorgesehen ist, mit dem nach Ablauf der Bestrahlungszeit die Lichtquelle (12) automatisch abschaltbar ist.

16. Lichtsystem nach einem der Ansprüche 1 bis 15, **dadurch gekennzeichnet, dass** die motorgestützte Positioniereinheit (30; 110) zumindest einen Linearmotor (112, 118, 126) aufweist.

17. Lichtsystem nach einem der Ansprüche 1 bis 16, **dadurch gekennzeichnet, dass** am distalen Ende (26; 70) des Lichtleiters (20; 64; 106) eine Schutzabdeckung (72) vorgesehen ist.

18. Lichtsystem nach Anspruch 17, **dadurch gekennzeichnet, dass** die Schutzabdeckung (72) sterilisierbar ist.

19. Lichtsystem nach Anspruch 17 oder 18, **dadurch gekennzeichnet, dass** die Schutzabdeckung (72) zur Einmalverwendung vorgesehen ist.

20. Lichtsystem nach einem der Ansprüche 1 bis 19, **dadurch gekennzeichnet, dass** es ein die Lichtquelle (12) enthaltendes Beleuchtungsmodul (62; 100) mit einer Steckeraufnahme (68; 102) aufweist, wobei ein proximales Ende (22; 66; 108) des Lichtleiters (20; 64; 106) abnehmbar in der Steckeraufnahme (68; 102) angeordnet ist.

## Claims

1. Light system for photodynamic diagnosis and/or therapy, with an incoherent light source (12) and with a light guide (20; 64; 106) which has an entrance interface (24), the entrance interface (24) and light source (12) being able to be positioned relative to one another, **characterized in that** the light system (10; 60) further comprises the following, namely a luminous power meter (32; 74) for measuring the luminous power emitted at a distal end (26; 70) of the light guide (20; 64; 106), a motorized positioning unit (30; 110) for positioning the entrance interface (24) and the light source (12) relative to one another, a control unit (36; 88) which controls the positioning unit (30; 110) as a function of the luminous power measured by the luminous power meter (32; 74) and an operating unit (50; 96) in the form of a remote control with which the luminous power at the distal end (26; 70) of the light guide (20; 64; 106) can be varied by the patient between predetermined values.

2. Light system according to claim 1, **characterized in that** the luminous power at the distal end (26; 70) of the light guide (20; 64; 106) can be maximized with the control unit (36; 88).

3. Light system according to claim 2, **characterized in that** the luminous power at the distal end (26; 70) of the light guide (20; 64; 106) is maximized by a gradient optimization method.

4. Light system according to any one of claims 1 to 3, **characterized in that** an IR block filter (41) is provided between the light source (12) and the light guide (20; 64; 106).

5. Light system according to claim 4, **characterized in that** a detector (46) is provided which detects radiation (44) reflected from the IR block filter (41).

6. Light system according to claim 5, **characterized in that** the power of the light source (12) can be controlled as a function of the radiation (44) detected by the detector (46).

7. Light system according to any one of claims 2 to 6, **characterized in that** the luminous power at the distal end (26; 70) of the light guide (20; 64; 106) is increased slowly to a maximum value.

8. Light system according to any one of claims 1 to 7, **characterized in that** the luminous power at the distal end (26; 70) of the light guide (20; 64; 106) is controlled by means of controlling the power of the light source (12).

9. Light system according to any one of claims 1 to 7, **characterized in that** the luminous power at the distal end (26; 70) of the light guide (20; 64; 106) is controlled by means of controlling the position of the entrance interface (24) of the light guide (20; 64; 106) and of the light source (12) relative to one another.

10. Light system according to any one of claims 1 to 9, **characterized in that** pulsed light can be emitted by the light source (12).

11. Light system according to claim 10, **characterized in that** a shutter (52) for generating pulsed light is provided on the light source (12).

12. Light system according to any one of claims 1 to 11, **characterized in that** an input unit is provided with which it is possible to define a radiation dose that is to be emitted from the distal end (26; 70) of the light guide (20; 64; 106).

13. Light system according to claim 12, **characterized in that** the input unit can be used to calculate and adjust an irradiation time as a function of the power of the light source (12), the position of the entrance interface (24) of the light guide (20; 64; 106) and of the light source (12) relative to one another, and the radiation dose that is to be emitted.

14. Light system according to claim 13, **characterized in that** the irradiation time is calculated continuously.

15. Light system according to any one of claims 1 to 14, **characterized in that** a timer (48) is provided with which the light source (12) can be automatically switched off after the irradiation time has elapsed.

16. Light system according to any one of claims 1 to 15, **characterized in that** the motorized positioning unit (30; 110) has at least one linear motor (112, 118, 126).

17. Light system according to any one of claims 1 to 16, **characterized in that** a protective cover (72) is provided at the distal end (26; 70) of the light guide (20; 64; 106).

18. Light system according to claim 17, **characterized in that** the protective cover (72) can be sterilized.

19. Light system according to claim 17 or 18, **characterized in that** the protective cover (72) is to be disposed of after use.

20. Light system according to any one of claims 1 to 19, **characterized in that** it comprises an illumination module (62; 100) containing the light source (12) and having a plug socket (68; 102), whereby a proximal end (22; 66; 108) of the light guide (20; 64; 106) is arranged removably in the plug socket (68; 102).

## Revendications

1. Système d'illumination pour le diagnostic et/ou la thérapie photodynamique, comportant une source de lumière (12) incohérente et un guide de lumière par fibres optiques (20 ; 64 ; 106) présentant une interface d'entrée (24), l'interface d'entrée (24) et la source de lumière (12) pouvant être positionnées l'une par rapport à l'autre, **caractérisé en ce que** le système d'illumination (10 ; 60) présente en outre les éléments suivants, à savoir
un dispositif de mesure de flux lumineux (32 ; 74) servant à mesurer le flux lumineux délivré à une extrémité distale (26 ; 70) du guide de lumière par fibres optiques (20 ; 64 ; 106),
une unité de positionnement assistée par moteur (30 ; 110) servant à positionner l'interface d'entrée (24) et la source de lumière (12) l'une par rapport à l'autre,
une unité de contrôle (36 ; 88), qui contrôle l'unité de positionnement (30 ; 110) en fonction du flux lumineux mesuré par le dispositif de mesure de flux lumineux (32 ; 74), et une unité de commande (50 ; 96) sous la forme d'une télécommande, grâce à laquelle un patient peut faire varier entre des valeurs prédéfinies le flux lumineux au niveau de l'extrémité distale (26 ; 70) du guide de lumière par fibres optiques (20 ; 64 ; 106).

2. Système d'illumination selon la revendication 1, **caractérisé en ce que** le flux lumineux peut être maximisé avec l'unité de contrôle (36 ; 88) au niveau de l'extrémité distale (26 ; 70) du guide de lumière par fibres optiques (20 ; 64 ; 106).

3. Système d'illumination selon la revendication 2, **caractérisé en ce qu'**un processus d'optimisation des gradients permet de maximiser le flux lumineux au niveau de l'extrémité distale (26 ; 70) du guide de lumière par fibres optiques (20 ; 64 ; 106).

4. Système d'illumination selon l'une quelconque des revendications 1 à 3, **caractérisé en ce qu'**un filtre de blocage infrarouge (41) est prévu entre la source de lumière (12) et le guide de lumière par fibres optiques (20 ; 64 ; 106).

5. Système d'illumination selon la revendication 4, **caractérisé en ce qu'**un détecteur (46) est prévu, lequel détecte le rayonnement (44) réfléchi par le filtre de blocage infrarouge (41).

6. Système d'illumination selon la revendication 5, **caractérisé en ce que** la puissance de la source de lumière (12) peut être contrôlée en fonction du rayonnement (44) détecté par le détecteur (46).

7. Système d'illumination selon l'une quelconque des revendications 2 à 6, **caractérisé en ce que** le flux lumineux peut être porté progressivement à une valeur maximale, au niveau de l'extrémité distale (26 ; 70) du guide de lumière par fibres optiques (20 ; 64 ; 106).

8. Système d'illumination selon l'une quelconque des revendications 1 à 7, **caractérisé en ce que** le contrôle de la puissance de la source de lumière (12) permet de contrôler le flux lumineux au niveau de l'extrémité distale (26 ; 70) du guide de lumière par fibres optiques (20 ; 64 ; 106).

9. Système d'illumination selon l'une quelconque des revendications 1 à 7, **caractérisé en ce que** le contrôle de la position de l'interface (24) du guide de lumière par fibres optiques (20 ; 64 ; 106) et de la source de lumière (12) l'une par rapport à l'autre permet de contrôler le flux lumineux au niveau de l'extrémité distale (26 ; 70) du guide de lumière par fibres optiques (20 ; 64 ; 106).

10. Système d'illumination selon l'une quelconque des revendications 1 à 9, **caractérisé en ce que** de la lumière pulsée peut être délivrée avec la source de lumière (12).

11. Système d'illumination selon la revendication 10, **caractérisé en ce qu'**un obturateur à diaphragme (52) servant à générer de la lumière pulsée est prévu au niveau de la source de lumière (12).

12. Système d'illumination selon l'une quelconque des revendications 1 à 11, **caractérisé en ce qu'**une unité d'entrée est prévue, laquelle permet de fixer une dose de rayonnement à délivrer par l'extrémité distale (26 ; 70) du guide de lumière par fibres optiques (20 ; 64 ; 106).

13. Système d'illumination selon la revendication 12, **caractérisé en ce que** l'unité d'entrée permet en fonction de la puissance de la source de lumière (12), de la position de l'interface d'entrée (24) du guide de lumière par fibres optiques (20 ; 64 ; 106) et de la source de lumière (12) l'une par rapport à l'autre et de la dose de rayonnement à délivrer, de calculer et de régler une durée d'irradiation.

14. Système d'illumination selon la revendication 13, **caractérisé en ce que** le calcul de la durée d'irradiation se fait de manière continue.

15. Système d'illumination selon l'une quelconque des revendications 1 à 14, **caractérisé en ce qu'**une minuterie (48) est prévue, laquelle permet d'éteindre automatiquement la source de lumière (12) à l'issue de la durée d'irradiation.

16. Système d'illumination selon l'une quelconque des revendications 1 à 15, **caractérisé en ce que** l'unité de positionnement (30 ; 110) assistée par moteur présente au moins un moteur linéaire (112, 118, 126).

17. Système d'illumination selon l'une quelconque des revendications 1 à 16, **caractérisé en ce qu'**un revêtement de protection (72) est prévu au niveau de l'extrémité distale (26 ; 70) du guide de lumière par fibres optiques (20; 64 ; 106).

18. Système d'illumination selon la revendication 17, **caractérisé en ce que** le revêtement de protection (72) peut être stérilisé.

19. Système d'illumination selon la revendication 17 ou 18, **caractérisé en ce que** le revêtement de protection (72) est prévu pour un usage unique.

20. Système d'illumination selon l'une quelconque des revendications 1 à 19, **caractérisé en ce qu'**il présente un module d'éclairage (62, 100) doté d'un logement de prise (68 ; 102) et contenant la source de lumière (12), une extrémité proximale (22 ; 66 ; 108) du guide de lumière par fibres optiques (20 ; 64 ; 106) étant disposée dans le logement de prise (68 ; 102) de manière amovible.
